# EUROPEAN PATENT SPECIFICATION

(11) **EP 3 206 659 B1**
(45) Date of publication and mention of the grant of the patent: **25.12.2019**
(21) Application number: 15780898.1
(22) Date of filing: 15.10.2015
(51) Int. Cl.: A61K 8/365, A61K 8/67, A61Q 17/04, A61Q 19/00, A61Q 19/08

(54) **USE OF A COSMETIC COMPOSITION COMPRISING 10-HYDROXYSTEARIC ACID**
KOSMETISCHE VERWENDUNG EINER ZUBEREITUNG ENTHALTEND 10-HYDROXYSTEARINSÄURE
UTILISATION COSMÉTIQUE D'UNE COMPOSITION CONTENANT DE L'ACIDE 10-HYDROXYSTÉARIQUE

(30) Priority: 17.10.2014 US 201462065102 P
(43) Date of publication of application: 23.08.2017
(73) Proprietor: DSM IP Assets B.V., 6411 TE Heerlen (NL)
(72) Inventor: IMFELD, Dominik, CH-4303 Kaiseraugst (CH); RADSPIELER, Alexander, CH-4303 Kaiseraugst (CH); SCHUETZ, Rolf, CH-4303 Kaiseraugst (CH)
(74) Representative: Berg, Katja
(86) International application number: PCT/EP2015/073912
(87) International publication number: WO 2016/059169

(56) References cited:
- EP-A2- 0 744 175
- WO-A1-2007/039057
- WO-A1-2014/016517
- WO-A1-2014/084676
- KR-B1- 100 693 292
- US-A- 4 424 234
- US-A- 5 747 051
- US-A1- 2010 286 102

## Description

### Technical Field

The present invention relates to the cosmetic use of topical compositions comprising 10-hydroxystearic acid, a salt or ester thereof, for application to human skin for improving the condition and appearance of skin more particularly as an anti-wrinkle, for promoting a homogenous skin tone, for reduction of skin pore size, as a photo-protecting agent, particularly to reduce the signs of skin photo damage and photo aging, and/or for improvement of the skin barrier function.

### Background of the invention

Human skin and especially facial skin is continuously exposed to tough conditions (external conditions of temperatures, hygrometry, UV light...) that all result in accelerating the natural skin aging process. In recent years, the demand for methods for improving the appearance and condition of skin has significantly increased calling for new and more efficient substances that are safe in use, and acting synergistically via different mode of action. As an example, EP1931329 discloses a broad range of hydroxy fatty acids that may have cosmetic effects in treating or preventing any symptoms caused by negative developments of the physiological homeostasis of healthy skin, as well as for the promotion of hair growth and protection from hair loss. However none of these compounds have been developed into commercial products because of the limited cosmetic effect when applied to human skin, because of the difficulty in establishing a commercial production for some of these compounds, and because of difficulties in establishing product forms in which the active remains soluble.

### Summary of the invention

The problem to be solved by the present invention is to find a hydroxy fatty acid which is safe in use, which acts as an antiaging cosmetic ingredient via multiple modes of action, which can easily be produced at industrial scale, and which can easily be formulated as a soluble ingredient in cosmetic compositions.

The present inventors surprisingly found that 10-hydroxystearic acid, a salt or ester thereof, solve all the above mentioned problems and can be used as a topical composition for application to human skin for improving the condition and appearance of skin more particularly as an anti-wrinkle, for promoting a homogenous skin tone, for reduction of skin pore size, as a photo-protecting agent, particularly to reduce the signs of skin photo damage and photo aging, and for improvement of the skin barrier function.

### Detailed description of the invention

Thus, in one aspect, the present invention is concerned with the cosmetic use of topical compositions comprising 10-hydroxystearic acid, a salt or ester thereof, for application to human skin for improving the condition and appearance of skin more particularly as an anti-wrinkle, for promoting a homogenous skin tone, for reduction of skin pore size, as a photo-protecting agent, particularly to reduce the signs of skin photo damage and photo aging, and for improvement of the skin barrier function.

10-hydroxystearic acid (CAS: 638-26-6) has the following formula:

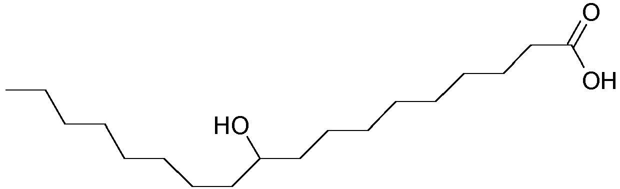

Both enantiomers may be used according to the present invention, and the preferred enantiomeric form is the (R)-10-hydroxystearic acid.

The ester is, preferably, an alkyl ester, wherein the term "alkyl" encompasses straight chain as well as branched alkyl groups, i.e. that "C₁₋₄-alkyl" encompasses straight chain C₁₋₄-alkyl (methyl, ethyl, n-propyl, n-butyl) as well as branched C₃₋₄-alkyl (iso-propyl, isobutyl, tert-butyl). Most preferred alky ester according to the present invention is a linear C₁₋₃ alkyl.

The salt may by formed by any cosmetically acceptable cation which means any metal cation as well as any organic cation that is not toxic to the skin and/or does not cause allergic reactions. Examples of such cations are ammonium salts and alkyl ammonium salts, alkali cations such as sodium and potassium ions and alkaline earth metal cations such as calcium and magnesium ions.

More specifically, the invention is concerned with the use of cosmetic compositions comprising 10-hydroxystearic acid, a salt or ester thereof, and optionally one retinoid, for improving the condition and appearance of skin more particularly as an anti-wrinkle, for promoting a homogenous skin tone, for reduction of skin pore size, as a photo-protecting agent, particularly to reduce the signs of skin photo damage and photo aging, and for improvement of the skin barrier function.

Retinoids for use according to the present invention are, e.g., retinoic acid or retinol and isomers, e.g. 9-, 11- or 13-cis isomers thereof, and derivatives thereof which comprise retinyl esters such as the acetate, phenylbutyrate, propanoate, laurate, palmitate, oleate, linoleate; or retinyl alkyl carbonate; or ethers such as retinoxytrimethylsilane; or (all trans)-retinal or its acetals; or retinoic acid or amides thereof such as methoxy PEG-12 retinamide ("PEG-12" = -(CH₂-CH₂-O-)₁₂)-

Improving the condition and appearance of skin comprises treatment or prevention of wrinkles or dry skin or sensitive skin, thickening of the epidermis, inhibition of senescence of skin cells, prevention or treatment of photodamage, prevention or treatment of oxidative stress phenomena, prevention or treatment of cellulite, prevention and treatment of disturbances in ceramide and lipid synthesis, prevention of excess sebum production, reduction of activities of matrix metallo proteases or other proteases in the skin, treatment and prevention of inflammatory skin conditions.

In still another aspect, the invention is concerned with a cosmetic method for improving the condition and appearance of skin more particularly as an anti-wrinkle, for promoting a homogenous skin tone, for reduction of skin pore size, as a photo-protecting agent, particularly to reduce the signs of skin photo damage and photo aging, and for improvement of the skin barrier function.

In all the embodiments of the present invention, the skin condition is preferably selected from reducing of skin pore size, and reducing of the appearance of wrinkles, or reducing of the appearance of wrinkles, or reduction of the signs of skin photo damage and photo aging.

A preferred embodiment of the present invention is a method of treating or preventing wrinkles, which method comprises the step of topically administering to a person in need of such effect an effective amount of 10-hydroxystearic acid or a salt, or ester as given above, optionally and preferably in combination with a retinoid.

A further object of the present invention is a method for the prevention or treatment of pigmentation disorders and/or for providing an even skin tone, which method comprises the step of topically administering to a person in need of such prevention or treatment an effective amount of a 10-hydroxystearic acid or a salt, or ester according to the present invention as defined above, wherein the composition contains a retinoid.

Another object of the present invention is a method for fortifying the pigmentation which method comprises the step of topically administering to a person in need of such fortification an effective amount of a 10-hydroxystearic acid or a salt, or ester according to the present invention as defined above and topically administering a tanning active, wherein the tanning active may be administered before, after or simultaneously with the administration of the effective amount of the composition according to the present invention, and wherein any retinoid is essentially absent in such a composition.

The term 'an effective amount' refers to an amount necessary to obtain a physiological effect. The physiological effect may be achieved by one application dose or by repeated applications. The dosage administered may, of course, vary depending upon known factors, such as the physiological characteristics of the particular composition comprising the fatty acid or a salt, ester or amide with the definitions and preferences as given above, optionally in combination with a retinoid and its mode and route of administration; the age, health and weight of the recipient; the nature and extent of the symptoms; the kind of concurrent treatment; the frequency of treatment; and the effect desired and can be adjusted by a person skilled in the art.

As stated above, a retinoid may be additionally present, providing an additive or synergistic cosmetic effect. Two or more retinoids may be present. If a retinoid is present in the compositions of the present invention, the ratio of the 10-hydroxystearic acid or a salt, or ester with the definitions and preferences as given above) to the retinoid is suitably from about 1000:1 to 1:1000, more preferably from about 100:1 to 1:100 and in particular from about 30:1 to 1:30 by weight. The fatty acids or a salt, or ester thereof as defined above show also an additive or synergistic effect with retinoids which are already present in the human skin.

In the compositions provided by the present invention, the amount of 10-hydroxystearic acid or a salt, or ester with the definitions and preferences as given above is suitably from about 0.0001 to about 50 %, preferably from about 0.001 to about 20 % by weight of the total composition. More preferably, the 10-hydroxystearic acid or a salt, or ester as defined above is contained in the composition in an amount of about 0.01 % by weight to about 1 % by weight, most preferably in an amount of about 0.5 % by weight. If a retinoid or derivative thereof is present, the amount of the latter is suitably from about 0.0001 to about 50 % by weight, preferably from about 0.001 to about 20 % by weight, most preferably in an amount of about 0.01 to about 1 weight%, in particular in an amount of about 0.1 % by weight, based on the total amount of the composition. Preferred according to the invention is a composition comprising the 10-hydroxystearic acid or a salt, or ester with the definitions and preferences as given above in an amount of about 0.5 % by weight and a retinoid or derivative thereof in an amount of about 0.1 % by weight, based on the total amount of the composition.

The term "cosmetic composition" as used herein e.g. refers to topical compositions for care of the human skin, see also the heading "Kosmetika" in Römpp Lexikon Chemie, 10th edition 1997, Georg Thieme Verlag Stuttgart, New York.

Regarding the kind of the topical compositions and the preparation of the topical compositions as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

The amount of the cosmetic composition according to the invention, which is to be applied to the skin depends on the concentration of the active ingredients in the compositions and the desired cosmetic or pharmaceutical effect. For example, application can be such that a creme is applied to the skin. A creme is usually applied in an amount of 2 mg creme/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

The cosmetic composition according to the invention is preferably applied at least once per day, e.g. twice or three times a day. Usually it takes at least two weeks until the desired effect is achieved. However, it can take several weeks or even months until the desired effect is fully maximized.

The compositions of the present invention contain the 10-hydroxystearic acid or a salt, or ester thereof with the definitions and preferences as defined above with cosmetically acceptable excipients or diluents. If nothing else is stated, the excipients, additives, diluents, etc. mentioned in the following are suitable for cosmetic compositions. If nothing else is stated, in this application parts and percentages are per weight and are based on the total weight of the composition.

Preferably, the cosmetic compositions of the present invention are topical compositions in the form of a suspension or dispersion in solvents or fatty substances, or alternatively in the form of an emulsion or micro emulsion (in particular of O/W or W/O type, O/W/O or W/O/W-type), PET-emulsions, multiple emulsions, bickering emulsions, hydrogels, alcoholic gels, lipogels, one or multiphase solutions or a vesicular dispersion and other usual compositions, which can also be applied by pens, as masks or as sprays. The emulsions can also contain anionic, nonionic, cationic or amphoteric surfactant(s).

Preferred compositions according to the invention are skin care preparations, hair-care preparations, decorative preparations, light protection preparations and functional preparations.

Examples of skin care preparations are, in particular, body oils, body lotions, body gels, treatment creams, skin protection ointments, shaving preparations, such as shaving foams or gels, skin powders such as baby powder, moisturizing gels, moisturizing sprays, revitalizing body sprays, cellulite gels, and peeling preparations.

Examples of hair care preparations are, for example, hair-washing preparations in the form of shampoos, hair conditioners, hair-care preparations, e. g. treatment preparations, pre-treatment preparations, hair tonics, styling creams, styling gels, pomades, hair rinses, treatment packs, intensive hair treatments, hair-straightening preparations, liquid hair-setting preparations, hair foams and hairsprays and lacquers, perming agents, hair gels, hair fixatives and hair dying or bleaching agents.

Examples of decorative preparations are in particular lipstick, eye shadow, mascaras, dry and moist make-up, rouge, powders, and suntan lotions.

Examples of functional preparations are cosmetic or pharmaceutical compositions containing active ingredients such as hormone preparations, vitamin preparations, vegetable extract preparations, anti-ageing preparations, and antimicrobial (antibacterial or antifungal) preparations without being limited thereto.

Cosmetic compositions in accordance with the invention can be in the form of a liquid, a lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foams, sprays, sticks, a gel, a plaster, a powder, a cleanser, a soap or aerosols or wipes.

The compositions of the invention can also contain usual cosmetic or pharmaceutical adjuvants and additives, such as preservatives/ antioxidants, fatty substances/ oils, water, organic solvents, silicones, thickeners, softeners, emulsifiers, sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into cosmetics or medicaments.

An additional amount of antioxidants/ preservatives is generally preferred. Based on the invention all known antioxidants usually formulated into cosmetics or medicaments can be used. Especially preferred are antioxidants chosen from the group consisting of amino acids (e.g. glycine, histidine, tyrosine, tryptophane) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, Na-ascorbylacetate), tocopherol and derivatives (such as vitamin-E-acetate), mixtures of natural or synthetic vitamin E, vitamin A and derivatives (vitamin-A-palmitate and -acetate) as well as coniferylbenzoat, rutinic acid and derivatives, α-glycosylrutin, ferulic acid, furfurylidenglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 weight% to about 10 weight% of the total weight of the composition of the present invention. Preferably, one or more preservatives/antioxidants are present in an amount about 0.1 weight% to about 1 weight%.

Typically topical formulations also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol® A), diethanolamine cetyl phosphate (Amphisol®), potassium cetyl phosphate (Amphisol® K), sodium glyceryl oleate phosphate, hydrogenated vegetable glyceride phosphates and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol® A), diethanolamine cetyl phosphate (Amphisol®), potassium cetyl phosphate (Amphisol® K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 weight% to about 20 weight% of the total weight of the composition of the present invention. Preferably, about 0.1 weight% to about 10 weight% of emulsifiers is used.

The lipid phase of the topical compositions can advantageously be chosen from: mineral oils and mineral waxes; oils such as triglycerides of caprinic acid or caprylic acid and castor oil; oils or waxes and other natural or synthetic oils, in a preferred embodiment esters of fatty acids with alcohols e.g. isopropanol, propylene glycol, glycerin or esters of fatty alcohols with carboxylic acids or fatty acids; alkylbenzoates; and/or silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical compositions of the present invention include polar oils such as lecithines and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefines, hydrogenated polyisobutenes and isohexadecanes, favored polyolefines are polydecenes; dialkyl ethers such as dicaprylylether; linear or cyclic silicone oils such as preferably cyclomethicones (octamethylcyclotetrasiloxane; cetyldimethicone, hexamethylcyclotrisiloxane, polydimethylsiloxane, poly(methylphenylsiloxane) and mixtures thereof.

Still other fatty components which can advantageously be incorporated in topical compositions of the present invention are isoeikosane; neopentylglykoldiheptanoate; propyleneglykoldicaprylate/ dicaprate; caprylic/ capric/ diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat/hexacaprate; propylenglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical composition of the present invention to maintain hydration or rehydrate the skin. Moisturizers that prevent water from evaporating from the skin by providing a protective coating are called emollients.

Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 weight% to about 20 weight% of the total weight of the composition. The preferred amount of emollient is about 2 weight% to about 15 weight%, and most preferably about 4 weight% to about 10 weight%.

Moisturizers that bind water, thereby retaining it on the skin surface are called humectants. Suitable humectants can be incorporated into a topical composition of the present invention such as glycerin, polypropylene glycol, polyethylene glycol, lactic acid, pyrrolidon carboxylic acid, urea, phopholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 weight% to about 8 weight% in a composition of the present invention, preferably about 1 weight% to about 5 weight%.

The aqueous phase of the preferred topical compositions of the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or-monobutylether, diethyleneglycol monomethyl-or monoethylether and analogue products, polymers, foam stabilisators; electrolytes and especially one or more thickeners.

Thickeners that may be used in formulations of the present invention to assist in making the consistency of a product suitable include carbomer, siliciumdioxide, magnesium and/ or aluminium silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole® of type 980, 981, 1382, 2984, 5984 alone or mixtures thereof.

Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 weight% to about 8 weight% in the composition of the present invention, preferably, 1 weight% to about 5 weight%.

The addition of electrolytes into the composition of the present invention may be necessary to change the behavior of a hydrophobic emulsifier. Thus, the emulsions/ microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 weight% to about 8 weight% in the composition of the present invention.

According to the invention for preparing the compositions of the invention the active ingredients can be used as such or in an encapsulated form, for example in a liposomal form. Liposomes are preferably formed with lecithins with or without addition of sterols or phytosterols. The encapsulation of the active ingredients can be alone or together with other active ingredients. It is possible to encapsulate only the fatty acid or a salt, ester or amide thereof with the preferences and definitions as defined above or only the retinoid, but it is also possible to encapsulate both ingredients either together or in separate capsules.

Other embodiments include solid or semisolid capsules aiming to protect the retinoid from degradation or for controlled delivery. The capsule may contain the retinoid alone or together with the fatty acid or a salt, ester or amide thereof with the preferences and definitions as defined above. Suitable encapsulation technologies are for example described in WO 0180823, WO 9903450, WO 9317784 or in Fragrance Journal (2001), 29(2), 83-90.

The composition of the present invention can also contain one or more additional pharmaceutically or cosmetically active ingredients, in particular for preventing or reducing acne, wrinkles, lines, atrophy, inflammation, as well as topical anesthetics, artificial tanning agents and accelerators, antimicrobial agents, antifungal agents and sun screening additives without being limited thereto.

Examples of such ingredients are peptides (e.g., Matrixyl™ [pentapeptide derivative]), oligopeptides, wax-based synthetic peptides (e.g., octyl palmitate and tribehenin and sorbitan isostearate and palmitoyl-oligopeptide), glycerol, urea, guanidine (e.g. amino guanidine); vitamins and derivatives thereof such as vitamin C (ascorbic acid), vitamin A (e.g., retinoid derivatives such as retinyl palmitate or retinyl propionate), vitamin E (e.g., tocopherol acetate), vitamin B₃ (e.g. niacinamide) and vitamin B₅ (e.g. panthenol), vitamin B₆ and vitamin B₁₂, biotin, folic acid; anti-acne actives or medicaments (e.g. resorcinol, salicylic acid, and the like); antioxidants (e.g. phytosterols, lipoic acid); flavonoids (e.g. isoflavones, phytoestrogens); skin soothing and healing agents such as aloe vera extract, allantoin and the like; agents suitable for aesthetic purposes such as essential oils, fragrances, skin sensates, opacifiers, aromatic compounds (e.g., clove oil, menthol, camphor, eucalyptus oil, and eugenol), desquamatory actives, hydroxy acids such as AHA acids, radical scavengers, farnesol, antifungal actives in particular bisabolol, alkyldiols such as 1,2-pentanediol, hexanediol or 1,2-octanediol, phytol, polyols such as phytanetriol, ceramides and pseudoceramides, amino acids, protein hydrolysates, polyunsaturated fatty acids, plant extracts like kinetin, DNA or RNA and their fragmentation products, carbohydrates, conjugated fatty acids, carnitin, carnosine, biochinonen, phytofluen, phytoen, and their corresponding derivatives.

Additionally the cosmetic and pharmaceutical topical composition of the present invention may contain UV-screening agents (UV-filter). The additional UV-screening agents are advantageously selected from IR, UV-A, UV-B, UV-C and/ or broadband filters. Examples of UV-B or broad spectrum screening agents, i.e. substances having absorption maximums between about 290 nm and 340 nm may be organic or inorganic compounds. Organic UV-B or broadband screening agents are e.g. acrylates such as 2-ethylhexyl 2-cyano-3,3-diphenylacrylate (octocrylene, PARSOL® 340), ethyl 2-cyano-3,3-diphenylacrylate and the like; camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as PARSOL® SLX; drometrizole trisiloxane (Mexoryl XL); imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL® EHS, Neo Heliopan OS), isooctyl salicylate or homomenthyl salicylate (homosalate, PARSOL® HMS, Neo Heliopan HMS) and the like; triazine derivatives such as ethylhexyl triazone (Uvinul T-150), diethylhexyl butamido triazone (Uvasorb HEB) and the like. Encapsulated UV-filters such as encapsulated ethylhexyl methoxycinnamate (Eusolex UV-pearls) or microcapsules loaded with UV-filters as e.g. dislosed in EP 1471995 and the like;

Examples of broad spectrum or UV A screening agents i.e. substances having absorption maximums between about 320 nm and 400 nm may be organic or inorganic compounds. Organic broad spectrum or UV A screening agents include e.g. dibenzoylmethane derivatives such as 4-tert.-butyl-4'-methoxydibenzoyl-methane (PARSOL® 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (Tinosorb M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-Diethylamino-2-hydroxy-benzoyl)-benzoic acid hexylester (Uvinul A plus) as described in the European Patent Publication EP 1046391; Ionic UV-A filters as described in the International Patent Publication WO2005080341 A1; As dibenzoylmethane derivatives have limited photostability it may be desirable to photostabilize these UV-A screening agents. Thus, the term "conventional UV-A screening agent" also refers to dibenzoylmethane derivatives such as e.g. PARSOL® 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

A good overview of UV-A- and UV-B screening agents which can be added to the compositions of the present invention can also be found in DE-A 103 27 432. All UV-screening agents disclosed in this document are also useful as components for the compositions of the present invention and are included herein by reference. Additionally the composition of the present invention may contain UV-A and UV-B filters. Further examples of UV- filters or screening agents are disclosed, e.g., in WO 04/000256, see especially pages 10-12 which are included herein by reference.

A safe and effective amount of the UV-screening agent is used, typically from about 1 wt.-% to about 20 wt.-%, more typically from about 2 wt.-% to about 10 wt.-% based on the total weight of the composition.

Other suitable UV-screening agents which may be incorporated into the cosmetic or pharmaceutical topical compositions of the present invention are inorganic pigments such as microparticulated metal oxides (e.g. PARSOL® TX). Examples of such compounds include e.g. titanium dioxide having an average primary particle size of from about 15 nm to about 100 nm, zinc oxide having an average primary particle size of from about 15 nm to about 150 nm, zirconium oxide having an average primary particle size of from about 15 nm to about 150 nm, iron oxide having an average primary particle size of from about 15 nm to about 500 nm, and mixtures thereof. The metal oxide particles may also be coated by metal oxides such as e.g. aluminum or zirconium oxides or by organic coatings such as e.g. polyols, methicone, aluminum stearate, alkyl silane. Such coatings are well known in the art. When used herein, the inorganic sunscreens are present in the amount of from about 0.1 wt.-% to about 20 wt.-%, preferably from about 0.5 wt.-% to about 10 wt.-%, more preferably from about 1wt.-% to about 5 wt.-% based on the total weight of the composition.

The 10-hydroxystearic acid, salt, or ester are known or belong to a known class of compounds and as such can be prepared by known methods or in analogy thereto.

The ability of the cosmetic compositions of the present invention to reduce skin wrinkles may be assessed by profilometric methods described in "Skin topography measurement by interference fringe projection: a technical validation". (Lagarde J M; Rouvrais C; Black D; Diridollou S; Gall Y, Skin research and technology: official journal of International Society for Bioengineering and the Skin (ISBS) [and] International Society for Digital Imaging of Skin (ISDIS) [and] International Society for Skin Imaging (ISSI) (2001 May), 7(2), 112-21 or "Direct and non-direct measurement techniques for analysis of skin surface topography". Fischer T W; Wigger-Alberti W; Elsner P., Skin pharmacology and applied skin physiology (1999 Jan-Apr), 12(1-2), 1-11.

The following examples exemplify the invention, but they should not be construed as limiting the invention.

### Examples

The present invention is further illustrated by the following experiments.

### Example 1

An anti-aging formulation containing the ingredients indicated below can be prepared in a manner known per se.

### Example formulation Nr. 1

| **Ingredients** | **% (w/w)** |
|---|---|
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Tocopheryl Acetate | 0.50 |
| Ethylhexyl Methoxycinnamate | 4.00 |
| Ethylhexyl Salicylate | 2.00 |
| Butyl Methoxydibenzoylmethane | 1.00 |
| Almond Oil | 2.00 |
| BHT | 0.05 |
| Phenoxyethanol & Methylparaben & Ethylparaben & Propylparaben & Butylparaben & Isopropylparaben | 0.80 |
| Disodium EDTA | 0.10 |
| D-Panthenol | 0.30 |
| Propylene Glycol | 4.00 |
| Polyacrylamide & C13-14 Isoparaffin & Laureth-7 | 0.50 |
| 10-hydroxy stearic acid | 0.50 |
| Water | Ad 100 |
| Triethanolamine | q.s. |

### Example 2

An anti-aging formulations containing the ingredients indicated below can be prepared in a manner known per se.

### Example formulation Nr. 2

| **Ingredients** | **% (w/w)** |
|---|---|
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Tocopheryl Acetate | 0.50 |
| Ethylhexyl Methoxycinnamate | 4.00 |
| Ethylhexyl Salicylate | 2.00 |
| Butyl Methoxydibenzoylmethane | 1.00 |
| Almond Oil | 2.00 |
| BHT | 0.05 |
| Phenoxyethanol & Methylparaben & Ethylparaben & Propylparaben & Butylparaben & Isopropylparaben | 0.80 |
| Disodium EDTA | 0.10 |
| D-Panthenol | 0.30 |
| Propylene Glycol | 4.00 |
| Polyacrylamide & C13-14 Isoparaffin & Laureth-7 | 0.50 |
| Water | Ad 100 |
| 10-hydroxy-stearic acid | 0.50 |
| Triethanolamine | q.s. |

### Example 3

A facial treatment formulation containing the ingredients indicated below can be prepared in a manner known per se

### Formulation Nr. 3

| **Ingredients** | **% (w/w)** |
|---|---|
| Glyceryl Myristate | 5.00 |
| Cetyl Alcohol | 2.00 |
| Cetyl Phosphate | 2.00 |
| Isopropyl Myristate | 10.00 |
| Tocopheryl Acetate | 0.30 |
| Almond Oil | 2.00 |
| BHT | 0.05 |
| Phenoxyethanol & Methylparaben & Ethylparaben & Propylparaben & Butylparaben & Isopropylparaben | 0.60 |
| Tromethamine | 0.90 |
| Water | Ad. 100 |
| D-Panthenol | 0.20 |
| Disodium EDTA | 0.10 |
| Propylene Glycol | 4.00 |
| Polyacrylamide & C13-14 Isoparaffin & Laureth-7 | 2.00 |
| 10-hydroxy stearic acid | 0.50 |
| Triethanolamine | q.s. |

### Example 4

A facial treatment formulation containing the ingredients indicated below can be prepared in a manner known per se

### Formulation Nr. 4

| **Ingredients** | **% (w/w)** |
|---|---|
| Glyceryl Myristate | 5.00 |
| Cetyl Alcohol | 2.00 |
| Cetyl Phosphate | 2.00 |
| Isopropyl Myristate | 10.00 |
| Tocopheryl Acetate | 0.30 |
| Almond Oil | 2.00 |
| BHT | 0.05 |
| Phenoxyethanol & Methylparaben & Ethylparaben & Propylparaben & Butylparaben & Isopropylparaben | 0.60 |
| Tromethamine | 0.90 |
| Water | Ad. 100 |
| D-Panthenol | 0.20 |
| Disodium EDTA | 0.10 |
| Propylene Glycol | 4.00 |
| Polyacrylamide & C13-14 Isoparaffin & Laureth-7 | 2.00 |
| 10-hydroxy stearic acid | 0.50 |
| Triethanolamine | q.s. |

### Example 5

Hair loss sera containing the ingredients indicated below can be prepared in a manner known per se

### Formulation Nr. 5

| **Ingredients** | **% (w/w)** |
|---|---|
| Water | Ad. 100 |
| Ethanol | 8.00 |
| Isopropanol | 4.00 |
| Propylene Glycol | 5.00 |
| D-Panthenol | 0.20 |
| PEG-12 Dimethicone | 0.20 |
| PEG-40 Hydrogenated Castor Oil | 4.00 |
| Phytantriol | 0.05 |
| Vitamin E Acetate | 0.10 |
| 10-hydroxy stearic acid | 0.50 |
| Retinol 15D (Caprylic/Capric Triglyceride & Retinol) | 0.50 |
| NaOH 10% | q.s. |

### Example 6

Hair loss sera containing the ingredients indicated below can be prepared in a manner known per se

### Formulation Nr. 6

| **Ingredients** | **% (w/w)** |
|---|---|
| Water | Ad. 100 |
| Ethanol | 8.00 |
| Isopropanol | 4.00 |
| Propylene Glycol | 5.00 |
| D-Panthenol | 0.20 |
| PEG-12 Dimethicone | 0.20 |
| PEG-40 Hydrogenated Castor Oil | 4.00 |
| Phytantriol | 0.05 |
| Vitamin E Acetate | 0.10 |
| 10-hydroxyy stearic acid | 0.50 |
| NaOH 10% | q.s. |

### Example 7

A skin fortifier lotion containing the ingredients indicated below can be prepared in a manner known per se

### Formulation Nr. 7

| **Ingredients** | **% (w/w)** |
|---|---|
| Water | Ad. 100 |
| D- Panthenol | 0.05 |
| Sodium Ascorbyl Phosphate | 0.20 |
| Propylene Glycol | 5.00 |
| Acrylates/C10-30 Alkyl Acrylate Crosspolymer | 0.50 |
| Sodium Hydroxide 30% | 0.40 |
| Disodium EDTA | 0.10 |
| Squalane | 2.00 |
| Phenoxyethanol & Methylparaben & Ethylparaben & Propylparaben & Butylparaben & Isopropylparaben | 0.80 |
| Coco-Caprylate/Caprate | 4.00 |
| BHT | 0.05 |
| Tocopheryl Acetate | 0.10 |
| Cyclomethicone | 3.00 |
| Glycerin | 3.00 |
| 10-hydroxy stearic acid | 0.50 |
| Sodium Hydroxide 10% | q.s. |

### Example 8

A skin fortifier lotion containing the ingredients indicated below can be prepared in a manner known per se

### Formulation Nr. 8

| **Ingredients** | **% (w/w)** |
|---|---|
| Water | Ad. 100 |
| D- Panthenol | 0.05 |
| Sodium Ascorbyl Phosphate | 0.20 |
| Propylene Glycol | 5.00 |
| Acrylates/C10-30 Alkyl Acrylate Crosspolymer | 0.50 |
| Sodium Hydroxide 30% | 0.40 |
| Disodium EDTA | 0.10 |
| Squalane | 2.00 |
| Phenoxyethanol & Methylparaben & Ethylparaben & Propylparaben & Butylparaben & Isopropylparaben | 0.80 |
| Coco-Caprylate/Caprate | 4.00 |
| BHT | 0.05 |
| Tocopheryl Acetate | 0.10 |
| Cyclomethicone | 3.00 |
| Glycerin | 3.00 |
| 10-hydroxy stearic acid | 0.50 |
| Sodium Hydroxide 10% | q.s. |

### Example 9

A formulation to treat age spots containing the ingredients indicated below can be prepared in a manner known per se

### Formulation Nr. 9

| **Ingredients** | **% (w/w)** |
|---|---|
| Water | Ad. 100 |
| Polyquaternium-10 | 0.10 |
| D-Panthenol | 0.50 |
| Sodium Ascorbyl Phosphate | 1.00 |
| Niacinamid | 0.50 |
| Propylene Glycol | 4.00 |
| Glycerin | 3.00 |
| PEG-12 Dimethicone | 0.20 |
| Disodium EDTA | 0.10 |
| Polysorbate 20 | 5.00 |
| Phenoxyethanol & Methylparaben & Ethylparaben & Propylparaben & Butylparaben & Isopropylparaben | 0.80 |
| 10-hydroxy stearic acid | 0.50 |
| Sodium Hydroxide 10% | q.s. |

### Example 10: Effect of 10-Hydroxystearic acid on collagen-I synthesis

The compounds were dissolved at 10 mM in DMSO, aliquoted and stored at -20°C.

The aim of this work was to test the efficacy of 10-Hydroxystearic acid (10-HSA) on Collagen 1 synthesis in human dermal fibroblasts.
Human dermal fibroblasts isolated from a biopsy of a 50 year old female donor (Fb-D) were maintained in Dulbecco's modified Eagle's medium high glucose (DMEM) containing 10% fetal calf serum (FCS) and 1% penicillin/streptomycin (P/S). Cells were cultivated at 37°C in a humidified 5% CO2-air atmosphere.

### Material and Methods.

### Cytotoxicity Measurement

Cytotoxicity was measured using the MTT assay according to the manufacturer's instructions. In brief: Fibroblasts (4000 cells/well, in in 96-well cell culture plates from Nunc) were pre-cultured in DMEM 10% FCS 1% Penicillin/Streptomycin (P/S) for 24 h before being starved in DMEM low glucose containing 0.2% FCS and 1% P/S for 2.5 days. Starvation medium was then replaced and solubilized compounds were added and incubated for 48 h. Subsequently 10 µl MTT labelling reagent were added to each well (final concentration 0.5 mg/ml) and the microtiter plate was incubated for 4 h (37°C, 5% CO2). After addition of 100 µl solubilization solution into each well, the plate was allowed to stand overnight in the incubator (37°C, 5% CO2). The absorbance (OD) was measured in an absorbance plate reader at 550 nm.

### Measurement of Collagen 1 Synthesis in Normal Human Fibroblasts

Human dermal fibroblasts were pre-cultured in DMEM 10% FCS 1% P/S for 24 h before being starved in DMEM low glucose containing 0.2% FCS and 1% P/S for 2.5 days. Starvation medium was then replaced and solubilized compounds were added and incubated for 48 h. After compound incubation cells were fixed in DPBS containing 4% formaldehyde for 15 min and permeabilized with DPBS 0.1% Triton-X100 for 90 sec. Collagen 1 was detected using mouse anti human collagen 1 antibody and the first antibody was detected with AlexaFluor 488 conjugated goat anti-mouse IgG. We counterstained nuclei with DAPI. Stained cells were analysed with the Thermo Fisher High Content Imaging System. There were acquired 49 pictures per well with a 10x objective and images were subsequently analysed using the "CellHealthProfiling" bioapplication v4. Collagen 1 was measured in a defined region around the nucleus and the fluorescence intensity values were normalized to cell count.

### Results:

### Cytotoxicity

Normal human fibroblasts treated with compounds for 48 h were assessed for viability using the MTT assay. Control values without compound were set to 100% viability. 10-HSA did not show any cytotoxic effect (reduction of cell survival to less than 80% compared to control cells).

### Collagen 1 synthesis

Following a pre-culture period, primary human fibroblasts were incubated for 48 h with increasing amounts of compound. Cells were then fixed and collagen 1 protein level was assessed by immunofluorescence. The experiment was repeated twice. After starvation fibroblasts were incubated for 48 h with increasing amounts of compound. TGF-β1 serves as positive control. Values given are fold collagen 1 induction compared with control cells. 10-HSA showed a dose dependent induction of collagen 1, while stearic acid (SA) did not induced collagen 1 expression. Collagen 1 induction reached 2.2 fold induction when 10-HSA was used at 5 µM.

### Example 11: Effect of 10-HSA on sun-burn cells and collagen 3

### Materials & Methods:

Skin sample: Human skin from abdominal plastic surgery has been used (donor: female, born in 1971). Informed consent has been obtained. The skin phototype has been assayed with a spectro-colorimeter and classified as "BROWN" (ITA° angle= 9,3°) (pigmentation scale: very light < light < intermediate < tanned < brown < dark) (Del Bino et al., Pigment Cell Research, 19. 2006).

Skin samples preparation: The skin samples have been cut in pieces of approximately 8x3 mm (Ø x thickness) and cultured up to day 6. Fourteen skin specimens have been used for each treatment. Two skin samples have been cultured to perform the skin viability test, six to perform the skin pigmentation and the collagen analyses and six for the SBC test.

Organ culture: Skin samples have been cultured in an air-liquid interface in a perforated ring of stainless steel in contact with culture medium (modified Williams' E medium) up to day 6. The culture medium has been renewed every three days (i.e. day 0-3).

Treatments: Both the test samples and the controls have been applied topically and renewed daily. The application has been performed as follows: skin biopsies have been gently cleaned with a cotton pad and 4µl of treatment for each tested sample has been applied on top of each piece and covered with a 7 Ø mm delivery membrane.

Skin viability: After 6 days of incubation two skin biopsies have been weighed and, in case, reduced in the dermal portion, in order to have approximately the same weight for all of them. Samples have then been washed and processed with Methylthiazolyldiphenyltetrazolium bromide (MTT). MTT is converted to water-insoluble dark blue colored MTT-formazan by mitochondrial dehydrogenases. Subsequently the blue crystals have been solubilized and the color intensity, that is directly proportional to skin vitality, has been measured with a plate reader at a wavelength of 570 nm. Being day 6 the endpoint of skin viability test, this analysis has not been performed on the samples stopped at day 2.

Sun-burn cells (SBC) quantification: SBC are keratinocytes undergoing apoptosis after they have received a physiological UVB dose that irreversibly and severely damages their DNA. This assay allows to investigate, following histochemical staining, the effect of a tested compound in preventing or mitigating DNA damage consequent to exposition to UVB light. Within this experimental procedure, haematoxylin-eosin staining is performed on skin sections. Twelve skin sections are obtained from the skin samples belonging to each treatment.

Collagen 3: Skin sections have been immune-stained with the selected antibody. The amount of the antigen present in each slide has been evaluated by estimating the intensity and the distribution of the red within a selected area of the dermis using Image J (NIH-USA). The selected antibody for collagen 3 has been the mouse monoclonal anti-Collagen 3 (Sigma cat#c7805).

Statistical analysis: All quantitative data have been summarized in terms of the mean score for each treatment. The measures of variation as standard deviation and standard error of mean (sem) have been applied to the original scores. Differences between groups were evaluated by One-way anova with permutation test followed by pairwise post-hoc comparisons - Dunnett's permutation test and pairwise post hoc comparisons - Tukey's HSD permutation tests.

### RESULTS:

SKIN VIABILITY: 10-HSA did not affect skin viability at any of the 3 concentrations tested (0.1, 1, and 10 mg/ml).

SUNBURN CELLS: As expected, UV irradiation has significantly increased the presence of sunburn cells (SBC) (+225%). The selected, commercially available, sun-protection cream (i.e. Eucerin 50+ Kids) has mitigated the formation of SBC (-25% vs untreated UV). 10-HSA has shown a protection on the UV-induced increase in SBC of 27 to 30%.

COLLAGEN 3: 10-HSA induced the synthesis of type 3 collagen. When tested at 0,1mg/ml and 1mg/ml 10-HSA has significantly increased the expression of collagen 3 (+49% and +57% respectively).

### Example 12: Effect of 10-HSA on MMP-1 modulation

Skin sample: Human skin from abdominal plastic surgery has been used (donor: male, born in 1948). Informed consent has been obtained. The skin phototype has been assayed with a spectro-colorimeter and classified as "LIGHT" (ITA° angle= 50°) (pigmentation scale: very light < light < intermediate < tanned < brown < dark) (Del Bino et al., Pigment Cell Research, 19. 2006).

Skin samples preparation: The skin samples have been cut in pieces of 8x3 mm (Ø x thickness) and cultured up to day 2. Two skin samples for each treatment have been cultured to perform the MMP-1 analysis.

Organ culture: Skin samples have been cultured in an air-liquid interface in a perforated ring of stainless steel in contact with culture medium (modified Williams' E medium) up to day 2. The culture medium has been renewed every three days (i.e. day 0-3).

Treatments: Both the test samples and the controls have been applied topically, renewed 1 hour prior of UVB irradiation. Right before the irradiation the treatments have been gently yet carefully removed from the skin samples. The application has been performed as follows: skin biopsies have been gently cleaned with a cotton pad and 4µl of treatment for each tested sample has been applied on top of each piece and covered with a 7 Ø mm delivery membrane.

UV Irradiation: Skin samples were UVB-irradiated (60 mJ/cm2) with a UVB light source (Bio-Link, Vilber Lourmat, Eberhardzell, Germany) equipped with UVB T8-M lamps (312 nm).

Quantification of the expression of MMP-1 by RT-qPCR: For RT-qPCR analysis, total RNA have been extracted from full thickness skin using RNAeasy mini kit for fibrous tissues (Qiagen) following manufacturer's instructions. After quantification, 400 ng of total RNA have been retro-transcribed using random hexamers and Superscript III (Invitrogen). The produced cDNA have been used to perform a Real Time PCR (SybrGreen protocol) with specific primers for the evaluation of MMP-1 expression. Ubiquitin and YWHAZ have been used as reference gene for data normalization.

Data acquisition and statistical analysis: RT-qPCR have been performed on RotorGene (Corbet lifescience) thermal cycler and REST 2009 V2.0.13 software (Qiagen) have been used for data analysis.

Results of the Analysis of MMP-1 expression: As expected the irradiation with UVB (1J/cm2) has significantly and dramatically enhanced the expression of MMP-1 (+438% vs untreated No UV). The commercially available selected UV filter has significantly inhibited the UV promoted activity (-45% vs untreated UV). 10-HSA has significantly and considerably reduced the expression of MMP-1 in comparison to their vehicle (i.e. DMSO).

### Example 13: Reduction of UV-induced stress marker p53

Cell culture was performed as in example 12 above.

p53 detection and quantification: Skin sections have been stained with the antibody for p53 (Abcam, #ab7757). The p53 positive cells have been counted and the obtained value have been divided upon the area covered by epidermis. Two slides of each skin sample have been processed by image acquisition and related analysis (i.e. 12 images for each test treatment).

Statistical analysis All quantitative data have been summarized in terms of the mean score for each treatment. The measures of variation as standard deviation and standard error of mean (SEM) have been applied to the original scores. Differences between groups were evaluated by One-way anova with permutation test followed by pairwise post-hoc comparisons - Dunnett's permutation test and pairwise post hoc comparisons - Tukey's HSD permutation tests.

Results p53: As expected, the irradiation with UVB (1J/cm2) has significantly and dramatically enhanced the stress marker p53 p53 (+8429% vs untreated No UV). The commercially available selected UV filter has significantly inhibited the UV promoted induction (-67% vs untreated UV). 10-HSA, when tested at 0,1 mg/ml, has exerted a protective action on p53 UV-induced stimulation (-46% vs DMSO UV).

### Example 14: Effect of 10-HSA on collagen 3 in human dermal fibroblasts Cell Culture:

Human dermal fibroblasts obtained from adult skin (50-year-old donor) were seeded into 96-well plates (4000 cells/well) and cultured in Dulbecco's Modified Eagle Medium (DMEM) with 10% fetal calf serum (FCS) and 1% Penicillin/Streptomycin (Life Technologies, Lucerne, Switzerland) at 37°C with 5% CO₂ for 24 h. After medium was changed with DMEM Low Glucose containing 0.2% FCS and 1% Pen/Strep, the cells were incubated for another 2.5 days. Then, starvation medium was replaced with fresh medium containing the test compounds. After 48 h incubation the cells were fixed in Dulbecco's Phosphate-Buffered Saline (DPBS) containing 4% formaldehyde (Life Technologies, Lucerne, Switzerland) for 15 min and permeabilized with 0.1% Triton-X100 in DPBS for 90 sec.

### Measurement of Collagen 3 Synthesis in Normal Human Fibroblasts:

Collagen 3 was detected using rabbit anti-human Type III collagen (BioTrend, Cologne, Germany), followed by the AlexaFluor 488 conjugated secondary IgG antibody (goat anti-rabbit IgG, Life Technologies, Lucerne, Switzerland). We counterstained the nuclei with 4',6-diamidino-2-phenylindole (DAPI) from Sigma-Aldrich (Buchs, Switzerland). Stained cells were analysed with the ArrayScan VTI HCS Reader (Thermo Fisher Scientific, Waltham, MA, USA). The total of 49 pictures per well was acquired with a 10x objective and images were subsequently analysed using the "CellHealthProfiling" bioapplication v4. Collagen was measured in a defined region around the nucleus and the fluorescence intensity values were normalized to the cell count. Data are based on minimum of two independent experiments.

### Results:

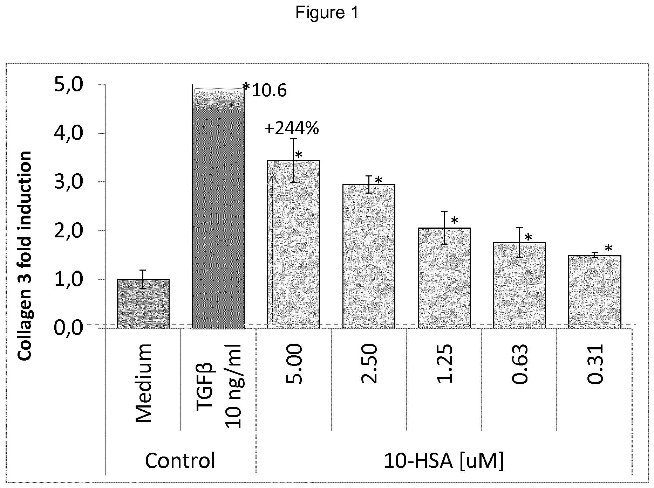

10-HSA significantly induced type III collagen synthesis in a dose-dependent manner in treated fibroblasts (* significance p < 0.01 relative to medium control). We observed an increase of 244 % in type III collagen with 10-HSA at a concentration of 5 µM compared with medium treated cells (Figure 1).

## Claims

1. Cosmetic non-therapeutic use of topical compositions comprising 10-hydroxystearic acid, a salt or ester thereof, for application to human skin for improving the condition and appearance of skin for reducing skin pore size, for reducing appearance of wrinkles, and/or for improvement of the skin barrier function.

2. Cosmetic non-therapeutic use according claim 1, wherein the improving the condition and appearance of skin is selected from reducing of skin pore size, and reducing of the appearance of wrinkles.

3. Cosmetic non-therapeutic use according claim 1, wherein the improving the condition and appearance of skin is selected from reducing of the appearance of wrinkles.

4. Composition comprising 10-hydroxystearic acid, a salt or ester thereof for use in improving the condition and appearance of skin, wherein said condition and appearance of skin is selected from reduction of the signs of skin photo damage and photo aging.

5. Cosmetic non-therapeutic use according to any of the claims 1 to 3 or composition for use according to claim 4, wherein the topical composition further comprises a retinoid.

6. Cosmetic non-therapeutic use or composition for use according to claim 5, wherein the retinoid is retinyl acetate, phenylbutyrate, propionate, octanoate, laurate, palmitate, oleate, linoleate; retinyl alkyl carbonate; retinoxytrimethylsilane; (all trans)-retinal or its acetals; or methoxy PEG-12 retinamide.

7. Cosmetic non-therapeutic use or composition for use according to any of the claims 1 to 6, wherein the amount of 10-hydroxystearic acid is from about 0.0001 to about 50 % by weight of the total composition.

8. Cosmetic non-therapeutic use or composition for use according to any of the claims 1 to 6, wherein the amount of 10-hydroxystearic acid is from 0.01 % by weight to 1 % by weight of the total composition.

9. Cosmetic non-therapeutic use or composition for use according to any of the claims 5 to 8, wherein the amount of the retinoid or derivative thereof is from about 0.001 to about 50 % by weight of the total composition.

10. Cosmetic non-therapeutic use or composition for use according to any of the claims 5 to 9, wherein the ratio of the 10-hydroxystearic acid to the retinoid or derivative thereof is from about 1000 : 1 to about 1 : 1000 by weight.

11. Cosmetic non-therapeutic use or composition for use according to any of claims 5 to 9, wherein the ratio the 10-hydroxystearic acid to the retinoid or derivative thereof is from about 30 : 1 to about 1 : 30 by weight.

12. A non-therapeutic method of cosmetic treatment for improving the condition and appearance of skin for reducing skin pore size, for reducing appearance of wrinkles, and for improvement of the skin barrier function, comprising applying to human skin a cosmetic composition comprising 10-hydroxystearic acid, a salt or ester thereof.

13. Non-therapeutic method according to claim 12, wherein the amount of 10-hydroxystearic acid is from 0.01 % by weight to 1 % by weight of the total composition.

14. Non-therapeutic method according to claim 12 or 13, wherein the cosmetic composition further comprises a retinoid.

15. Non-therapeutic method according to claim 14, wherein the ratio the 10-hydroxystearic acid to the retinoid or derivative thereof is from about 30 : 1 to about 1 : 30 by weight.

## Patentansprüche

1. Kosmetische nichttherapeutische Verwendung topischer Zusammensetzungen, die 10-Hydroxystearinsäure, ein Salz oder einen Ester davon umfassen, zum Aufbringen auf menschliche Haut zur Verbesserung des Zustands und des Erscheinungsbilds der Haut zur Verkleinerung der Porengröße der Haut, zur Reduktion des Auftretens von Falten und/oder zur Verbesserung der Barrierefunktion der Haut.

2. Kosmetische nichttherapeutische Verwendung nach Anspruch 1, wobei die Verbesserung des Zustands und des Erscheinungsbilds der Haut aus einer Verkleinerung der Porengröße der Haut und der Reduktion des Auftretens von Falten ausgewählt ist.

3. Kosmetische nichttherapeutische Verwendung nach Anspruch 1, wobei die Verbesserung des Zustands und des Erscheinungsbilds der Haut aus der Reduktion des Auftretens von Falten ausgewählt ist.

4. Zusammensetzung, die 10-Hydroxystearinsäure, ein Salz oder einen Ester davon umfasst, zur Verwendung zur Verbesserung des Zustands und des Erscheinungsbilds der Haut, wobei der Zustand und das Erscheinungsbild der Haut aus der Reduktion von Anzeichen lichtbedingter Hautschäden und lichtbedingter Hautalterung ausgewählt sind.

5. Kosmetische nichttherapeutische Verwendung nach einem der Ansprüche 1 bis 3 oder Zusammensetzung zur Verwendung nach Anspruch 4, wobei die topische Zusammensetzung weiterhin ein Retinoid umfasst.

6. Kosmetische nichttherapeutische Verwendung oder Zusammensetzung zur Verwendung nach Anspruch 5, wobei es sich bei dem Retinoid um Retinylacetat, -phenylbutyrat, -propionat, -octanoat, -laurat, -palmitat, -oleat, -linoleat; Retinylalkylcarbonat; Retinoxytrimethylsilan; all-trans-Retinal oder Acetale davon oder PEG-12-Methoxyretinamid handelt.

7. Kosmetische nichttherapeutische Verwendung oder Zusammensetzung zur Verwendung nach einem der Ansprüche 1 bis 6, wobei die Menge an 10-Hydroxystearinsäure etwa 0,0001 bis etwa 50 Gew.-%, bezogen auf das Gewicht der gesamten Zusammensetzung, beträgt.

8. Kosmetische nichttherapeutische Verwendung oder Zusammensetzung zur Verwendung nach einem der Ansprüche 1 bis 6, wobei die Menge an 10-Hydroxystearinsäure 0,01 Gew.-% bis 1 Gew.-%, bezogen auf das Gewicht der gesamten Zusammensetzung, beträgt.

9. Kosmetische nichttherapeutische Verwendung oder Zusammensetzung zur Verwendung nach einem der Ansprüche 5 bis 8, wobei die Menge des Retinoids oder Derivats davon etwa 0,001 bis etwa 50 Gew.-%, bezogen auf das Gewicht der gesamten Zusammensetzung, beträgt.

10. Kosmetische nichttherapeutische Verwendung oder Zusammensetzung zur Verwendung nach einem der Ansprüche 5 bis 9, wobei das Verhältnis der 10-Hydroxystearinsäure zu dem Retinoid oder Derivat davon etwa 1000:1 bis etwa 1:1000, bezogen auf das Gewicht, beträgt.

11. Kosmetische nichttherapeutische Verwendung oder Zusammensetzung zur Verwendung nach einem der Ansprüche 5 bis 9, wobei das Verhältnis der 10-Hydroxystearinsäure zu dem Retinoid oder Derivat davon etwa 30:1 bis etwa 1:30, bezogen auf das Gewicht, beträgt.

12. Nichttherapeutisches Verfahren zur kosmetischen Behandlung zur Verbesserung des Zustands und des Erscheinungsbilds der Haut zur Verkleinerung der Porengröße der Haut, zur Reduktion des Auftretens von Falten und zur Verbesserung der Barrierefunktion der Haut, umfassend das Aufbringen einer kosmetischen Zusammensetzung, die 10- Hydroxystearinsäure, ein Salz oder einen Ester davon umfasst, auf die menschliche Haut.

13. Nichttherapeutisches Verfahren nach Anspruch 12, wobei die Menge an 10-Hydroxystearinsäure 0,01 Gew.-% bis 1 Gew.-%, bezogen auf das Gewicht der gesamten Zusammensetzung, beträgt.

14. Nichttherapeutisches Verfahren nach Anspruch 12 oder 13, wobei die kosmetische Zusammensetzung weiterhin ein Retinoid umfasst.

15. Nichttherapeutisches Verfahren nach Anspruch 14, wobei das Verhältnis der 10-Hydroxystearinsäure zu dem Retinoid oder Derivat davon etwa 30:1 bis etwa 1:30, bezogen auf das Gewicht, beträgt.

## Revendications

1. Utilisation cosmétique non thérapeutique de compositions topiques comprenant de l'acide 10-hydroxystéarique, un sel ou ester de celui-ci, pour l'application sur de la peau humaine pour l'amélioration de l'état et de l'aspect de la peau pour la réduction de la taille des pores de la peau, pour la réduction de l'apparition de rides et/ou pour l'amélioration de la fonction de barrière de la peau.

2. Utilisation cosmétique non thérapeutique selon la revendication 1, l'amélioration de l'état et de l'aspect de la peau étant choisie entre la réduction de la taille des pores de la peau et la réduction de l'apparition de rides.

3. Utilisation cosmétique non thérapeutique selon la revendication 1, l'amélioration de l'état et de l'aspect de la peau étant choisie entre la réduction de l'apparition de rides.

4. Composition comprenant de l'acide 10-hydroxystéarique, un sel ou ester de celui-ci, destinée à être utilisée en amélioration de l'état et de l'aspect de la peau, lesdits état et aspect de la peau étant choisis entre la réduction des signes de photoendommagement et de photovieillissement de la peau.

5. Utilisation cosmétique non thérapeutique selon l'une quelconque des revendications 1 à 3 ou composition destinée à être utilisée selon la revendication 4, la composition topique comprenant en outre un rétinoïde.

6. Utilisation cosmétique non thérapeutique ou composition destinée à être utilisée selon la revendication 5, le rétinoïde étant l'acétate, le phénylbutyrate, le propionate, l'octanoate, le laurate, le palmitate, l'oléate, le linoléate de rétinyle ; un carbonate d'alkyle et de rétinyle ; le rétinoxytriméthylsilane ; le rétinal tout trans ou ses acétals ; ou le méthoxy-PEG-12-rétinamide.

7. Utilisation cosmétique non thérapeutique ou composition destinée à être utilisée selon l'une quelconque des revendications 1 à 6, la quantité d'acide 10-hydroxystéarique étant d'environ 0,0001 à environ 50 % en poids de la composition totale.

8. Utilisation cosmétique non thérapeutique ou composition destinée à être utilisée selon l'une quelconque des revendications 1 à 6, la quantité d'acide 10-hydroxystéarique étant de 0,01 % en poids à 1 % en poids de la composition totale.

9. Utilisation cosmétique non thérapeutique ou composition destinée à être utilisée selon l'une quelconque des revendications 5 à 8, la quantité du rétinoïde ou de dérivé de celui-ci étant d'environ 0,001 à environ 50 % en poids de la composition totale.

10. Utilisation cosmétique non thérapeutique ou composition destinée à être utilisée selon l'une quelconque des revendications 5 à 9, le rapport de l'acide 10-hydroxystéarique au rétinoïde ou au dérivé de celui-ci étant d'environ 1000:1 à environ 1:1000 en poids.

11. Utilisation cosmétique non thérapeutique ou composition destinée à être utilisée selon l'une quelconque des revendications 5 à 9, le rapport de l'acide 10-hydroxystéarique au rétinoïde ou au dérivé de celui-ci étant d'environ 30:1 à environ 1:30 en poids.

12. Méthode non thérapeutique de traitement cosmétique pour l'amélioration de l'état et de l'aspect de la peau pour la réduction de la taille des pores de la peau, pour la réduction de l'apparition de rides et pour l'amélioration de la fonction de barrière de la peau, comprenant l'application sur de la peau humaine d'une composition cosmétique comprenant de l'acide 10-hydroxystéarique, un sel ou ester de celui-ci.

13. Méthode non thérapeutique selon la revendication 12, la quantité d'acide 10-hydroxystéarique étant de 0,01 % en poids à 1 % en poids de la composition totale.

14. Méthode non thérapeutique selon la revendication 12 ou 13, la composition cosmétique comprenant en outre un rétinoïde.

15. Méthode non thérapeutique selon la revendication 14, le rapport de l'acide 10-hydroxystéarique au rétinoïde ou au dérivé de celui-ci étant d'environ 30:1 à environ 1:30 en poids.
